# EUROPEAN PATENT SPECIFICATION

(11) **EP 1 959 855 B1**
(45) Date of publication and mention of the grant of the patent: **04.06.2014**
(21) Application number: 06838670.5
(22) Date of filing: 30.11.2006
(51) Int. Cl.: A61N 5/06, A61K 9/00, A61B 18/24, A61B 18/00, A61B 17/00, A61K 9/127, A61K 41/00

(54) **BENIGN PROSTATE HYPERPLASIA TREATMENT USING PHOTOSENSITIZER AND LASER ABLATION**
BEHANDLUNG VON BENIGNER PROSTATAHYPERPLASIE MIT EINEM FOTOSENSIBILISATOR UND LASERABLATION
TRAITEMENT D'UNE HYPERTROPHIE BENIGNE DE LA PROSTATE AU MOYEN D'UN PHOTOSENSIBILISANT ET DE L'ABLATION LASER

(30) Priority: 01.12.2005 US 741285 P; 14.12.2005 US 750427 P; 13.11.2006 US 598401
(43) Date of publication of application: 27.08.2008
(73) Proprietor: Biolitec Pharma Marketing Ltd., F.T. Labuan (MY)
(72) Inventor: NEUBERGER, Wolfgang, 87000 F.T. Labuan (MY)
(74) Representative: Herrmann, Franz
(86) International application number: PCT/US2006/045822
(87) International publication number: WO 2007/064783

(56) References cited:
- WO-A1-92/10142
- WO-A1-2005/077457
- US-A- 5 322 507
- US-A- 5 416 878
- US-A- 5 454 782
- US-A- 5 713 847
- US-A- 5 713 847
- US-A1- 2003 073 908
- US-A1- 2003 130 575
- US-A1- 2003 229 131
- US-A1- 2004 002 647

## Description

### Background of the Invention

### 2. Field of the invention:

The present invention relates generally to the field of medical treatment using lasers and photosensitizer for selective, efficient vaporization, coagulation and destruction of unwanted body tissue, more particularly, coagulating/destruction of excess tissues in prostate gland beyond the depth of laser ablation while minimizing damage to adjoining tissue.

### 3. Invention Disclosure Statement

Benign Prostatic Hyperplasia (BPH) is a condition most commonly affecting men older than 50 years.. The prostate is a small ovoid gland located below the bladder and at the base of the penis. Part of the urethra passes through the middle of the prostate gland. As men age, the prostate gland enlarges due to growth of tissue obstructing urine flow through the urethra. Men with BPH have difficulties with urination, such as interrupted/weak flow of urine, and the need to urinate often. BPH condition is treated by both medicines and by surgical methods. Medicinal therapy for treating milder symptoms of BPH consists of 3 classes of agents, which includes alpha-andrenergic blockers, 5aR inhibitors and phytotherapeutics. These drugs provide relief from symptomatic BPH, and need to be taken daily. In the long-term they are not very effective, and are known to cause dizziness, fainting, decrease in blood pressure, impotency and other problems.

BPH is often treated by surgical removal of excess prostate tissue constricting the urethra, thus relieving the urethral obstruction and the associated problems. Generally used, standard surgical treatments include Transurethral Resection of the Prostate (TURP) which offers a patient the most rapid and long term relief. Other methods are open procedures such as retropubic prostatectomy, suprapubic prostatectomy, etc. All these surgical procedures are invasive and are associated with a number of side effects which include long hospitalization, prolonged bleeding, infection, impotence, retrograde ejaculation and short or long term incontinence.

Other less invasive procedures used in recent years include TransUrethral Needle Ablation procedure (TUNA) which uses low-level radiofrequency energy to ablate prostate tissue which is performed under local anesthesia. This procedure is not effective for treating middle lobe tissue and the other drawback is a long catheterization period and also requires re-treatment. A few other examples of less invasive surgery include TransUrethral Incision of Prostate (TUIP), TransUrethral electro Vaporization (TUVP), Transurethral Microwave Thermotherapy (TUMT) and laser therapy.

Radiofrequency, microwaves, acoustical and laser energies are used for prostate tissue ablation (thermotherapy method). These energies are conducted to the enlarged prostate tissue using a catheter which is inserted through the urethra. During these procedures severe trauma is often sustained by duct wall and surrounding tissues in the regions, this is reduced to some extent by using cooling systems in the device. In these procedures healthy tissues surrounding the prostate region are often indiscriminately destroyed causing loss of prostate function. Interstitial tissue ablation using high power laser and other light sources is also practiced for tissue ablation; these procedures can be performed in less time avoiding general anesthesia. Patients treated with interstitial coagulation can experience dysuria and discomfort for a short time after procedure.

U.S. Patent 6,554,824 by Davenport et al. describes a method for treating BPH using solid-state lasers emitting light with a wavelength from about 200 to about 1000 nm. The laser is operated in a long duration pulse mode to char the targeted prostate tissue. In this method the treatment site needs to be cooled to avoid unwanted tissue destruction due to the high temperature rise. The laser's energy is used to thermally degrade tissue by generating the denaturing temperature in the tissue and fluid surrounding the region of treatment. The extent of tissue ablation and destruction achieved depends on the power and wavelength of the laser beam used.

The above methods use high energy sources like lasers and other energy sources to thermally coagulate and/or destroy the excess prostate tissue. This can lead to irreversible cellular damage beyond the area of excess tissue due to the need for high temperatures, the extended temperature effect as the temperature drops off in tissue away from the actually irradiated tissue and the long thermal exposures. High temperatures are associated with tissue burning, carbonization and tissue disruption. However, in these instances hemostatis is less complete and vaporization of sufficient volumes of prostate tissue is relatively slow and inefficient.

A non-thermal laser method is PhotoDynamic Therapy (PDT), which involves the use of a suitable wavelength to activate previously administered photosensitizer to bring about a photocytotoxic effect. In publication U.S. 2002/0193850A1 by Selman, a method for diagnosing or treating prostatic disorders and related symptoms is disclosed. In this invention the PDT method is used either alone or in combination with hyperthermic therapy. The addition of thermal treatment gives rise to the problems detailed earlier. For PDT alone, the method calls for sensitizing prostatic tissue with an effective amount of photosensitizer followed by irradiating the sensitized tissue with a light source for a predetermined time and intensity to cause destruction in the tissue. PDT can cause initial swelling of the treated tissue, which can lead to closure of the channel until the necrotized tissue is removed. Used alone, PDT can be slow, as the destroyed tissue must be removed and generally additional treatments need to be applied to treat deeper diseased areas than can be reached by the wavelength and power available.

In the prior art, generally either PDT or thermotherapy are used alone for treating BPH. These procedures require long times and generally repeated treatments, and can cause the prior mentioned side effects. Hence, there is a need for a treatment method and device for effective alleviation of BPH symptoms with maximum diseased tissue removal and minimum complications.

A desirable treatment method, therefore, would be one which can selectively ablate the extraneous prostatic tissue without excessively affecting the urethra, urethral sphincter and the other surrounding healthy tissue; and which can, if desired, treat deeper excess tissue with minimal thermal damage beyond the diseased tissue. The present invention provides such methods of effective laser ablation and prostate tissue coagulation using a photosensitizer and laser in combination. This method can bring about complete destruction of the hyperplasic prostate tissue with reduced or no hospitalization and faster recovery with minimal side effects compared to that reported for prior art methods.

### Objectives and Brief Summary of the Invention

It is an objective of the present invention to provide a minimally invasive method of treatment, utilizing a Photosensitizer (PS) and laser ablation for an enhanced, fast, efficient treatment of Benign Prostate Hyperplasia (BPH).

It is one of the objectives of the present invention to increase the depth of the destruction process, which is more directional and results in an increased total lesion volume, thereby improving long-term results.

It is another objective of the present invention to reduce the treatment time sufficiently to eliminate the need to use general anesthesia on the patient for the treatment.

It is yet another objective of the present invention to reduce the need for multiple treatments to achieve the desired results.

It is also an objective of the present invention to provide wherever necessary one or more wavelengths simultaneously in combination with photosensitizer to achieve more effective tissue ablation in the prostatic region than with a laser alone.

It is still another objective of the present invention to use a suitable delivery system for the photosensitizer which includes but is not limited to liposomal, pegylated, or other novel formulations.

Briefly stated, a method and apparatus are disclosed to employ a combination of laser ablation/coagulation and photosensitizers (PS) to treat BPH. According to this method a photosensitizer is administered locally to the prostate zone, which is to be treated, using a suitable device to ensure an adequately uniform distribution of the photosensitizer in the hyperplasic prostate tissue to be treated. In one embodiment, enlarged prostate tissue is laser irradiated using an optical fiber inserted through an endoscope to ablate and coagulate tissue nearby and to activate the photosensitizer to destroy tissue deeper into the prostate. In another embodiment, a suitable laser wavelength is used to photoactivate the photosensitizer in the presensitized prostate tissue to enhance ablation, photo-coagulation and destruction of diseased prostate tissues. Additional wavelengths may be employed that would target different areas of the prostate. Generally, laser ablation is done in pulsed or continuous irradiations where the photosensitizer can be activated before, during, or after this radiation. Multiple wavelengths/laser sources can also be used to achieve laser ablation, photo-coagulation, and absorption by the photosensitizer. The photosensitizer used in the invention may be formulated in a liposomal or other drug carrier system to achieve an enhanced effect of the drug.

The above and other objects, features and advantages of the present invention will become apparent from the following description read in conjunction with the accompanying drawings. The invention is defined in the claims.

Other embodiments are presented for illustrative purposes only.

### Brief Description of Figures

Figure 1 illustrates a representation of an applicator needle system for drug delivery to the prostate and tissue therein of present invention.
Figure 2 illustrates the irradiation of the prostate tissue transurethrally using an optical fiber through an endoscope 205 to irradiate the prostate 203.

### Detailed Description of Preferred Embodiments

The present invention describes methods and devices which employ photosensitizers (PS) in combination with laser coagulation and ablation to treat Benign Prostate Hyperplasia (BPH) which normally cannot be treated as quickly and as efficiently using presently known methods. The methods and devices described herein can achieve coagulation and photo-cytotoxic effects to larger and deeper areas of the enlarged prostate tissue and also provide more complete and uniform destruction of the tissue with minimal thermal damage to adjacent normal tissue because the methods described herein do not rely solely on thermal effects to achieve these goals. The present invention is directed to meet these needs.

In this treatment method a suitably formulated Photosensitizer is injected into the prostate tissue directly. After a short pause for the PS to penetrate the tissue, the area is irradiated with one or more wavelengths that can be absorbed by the PS, ensuring quicker ablation in a substantially blood free environment due to deeper and larger area of tissue destruction. The initial tissue ablation is achieved quicker with the laser as the absorption of the prostate tissue is considerably enhanced by the presence of the photosensitizer.

The present invention helps to induce specific destruction of prostate tissue in a more directional manner with increased total lesion depth. In this new approach, deeper coagulation of prostate tissue is achieved using photoactivated photosensitizer which acts beyond the ablation depth; caused by the laser. In one embodiment, the laser energy used in the procedure has at least a dual role: (i) to ablate the tissue, and (ii) to be absorbed by the photosensitizer to cause tissue destruction. The activated photosensitizer can also continue to work even after irradiation has stopped.

The energy requirement may be substantially less than is required in isolated laser ablation and vaporization techniques. Blood loss during the procedure may be less giving a clearer field to work and diminishing complications. Especially beneficial is to have photo-coagulation by one of the laser wavelengths. Coagulation is associated with good hemostatis and requires less energy.

The treatment methods presented in this invention have other potential advantages. A major patient and doctor benefit is that the shorter treatment time can eliminate the requirement of general anesthesia, which in turn provides faster patient recovery and lower risk of post treatment complications.

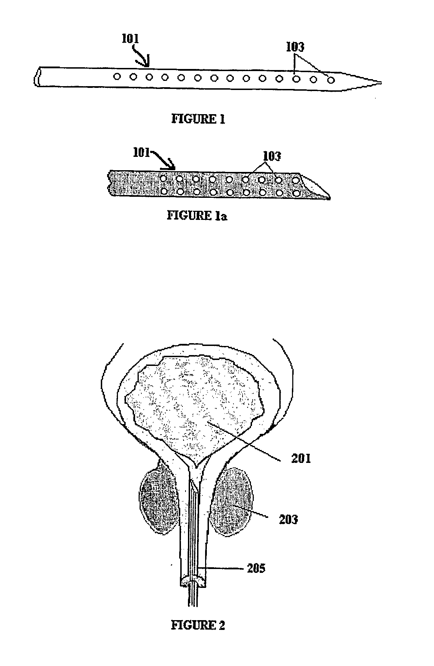

In the present invention, treatment for Benign Prostate Hyperplasic tissue involves: injecting the prostate tissue with a photosensitizing agent or its derivatives, suitably formulated in a carrier system which comprises, but is not limited to, a liposomal, pegylated, and other novel formulation. Any appropriate photosensitizing agent may be used, including for example chlorins or bacteriochlorins, including temoporfin. A measured dosage of the photosensitizer is injected into the prostate tissue directly using a special applicator needle system. As shown in Fig. 1, this applicator needle 101 has multiple holes 103 in its circumference, spaced for instance less than 3 mm apart along its axis, so as to ensure a good distribution of the active ingredient in the prostate tissue to be treated. After administration, a predetermined period of time is allowed to pass in order for photosensitizer to penetrate and distribute in the prostate hyperplasic tissue; this is followed by irradiation of the photosensitizer using light of a wavelength which overlaps the absorption spectrum of the administered photosensitizer. This initiates a sequence of photochemical, chemical and biological reactions, in addition to laser ablation, which ultimately lead to cell death in an extended area having the photosensitizer therein. The necrotized cells, beyond the ablation dimensions, are later sloughed from the body. The light is delivered to the prostatic tissue using an optical fiber inserted through an endoscope in the urethral canal. The optical fiber, a side firing fiber, is generally used to irradiate tissue, to avoid damage to sphincter muscles from the radiation. The irradiation light is transmitted substantially perpendicular to the axis of the optical fiber. The endoscope helps to introduce and correctly position the optical fiber within the urethra to irradiate the benign hyperplasic prostate tissue.

Within a short duration of irradiating the region, a significant volume of prostatic tissue is ablated or otherwise destroyed. The activation of the photosensitizer in the tissue will result in the killing of prostatic tissue in an additional zone of 2 to 3 mm depth or more, beyond the ablation and coagulation zone achieved by the laser irradiation alone, in the following days and weeks resulting in significantly greater elimination of prostatic tissue than simply by ablation. In this method, therefore, the actual treatment time is reduced compared to all other currently known treatment modalities. Hence the rate of tissue reduction is increased by the enhanced laser ablation and the post irradiation processes. Furthermore the procedure does not require general anesthesia and can therefore be carried out in an office setting.

In another embodiment, further enhancement of tissue ablation to eliminate deeper benign prostatic hyperplasia tissue uses a second laser wavelength that can be utilized simultaneously and concurrently with the first laser radiation. By means of selecting different wavelengths, diode laser sources can be selected for ablation/coagulation and for deeper photosensitizer activation for use concurrently or to target different zones with each different wavelength (e.g. side lobes: thermal ablation; central lobes: PDT).

Yet another embodiment of this invention is to use two or more different wavelength as suitable to photo-coagulate and to ablate tissue in the different areas of prostate tissue while another wavelength source simultaneously or substantially concurrently activates a photosensitizer.

While photodynamic therapy often initially results in some swelling of the treated tissue which is then followed by later destruction and elimination. The concurrent laser ablation, as performed in this invention, synergistically helps to create and maintain space for the passage of urine by removing immediately the hyperplasic tissue encroaching the urethra and thus this potential problem is not present when using this invention.

The present invention is further illustrated by the following examples, but is not limited thereby.

### Example 1: Laser ablation and photo-destruction at one wavelength

A liposomal temoporfin solution is administered by locally injecting 0.5 micrograms per gram of the prostate weight to be treated. (Thus for instance 25 micrograms is injected in a suitable solution if 50 g of prostate should be treated.) The injection can be done by utilizing a suitable needle with multiple holes in its circumference, (as shown for example in Figure 1) spaced for instance less than 3 mm apart along its axis, so as to ensure a good distribution of the active ingredient in the prostatic tissue to be treated. About 10 minutes after injection, a KTP laser (532nm), for example, set at about 80 Watts power level is used to ablate the prostatic tissue utilizing side firing fiber optics inserted through an endoscope as well as to activate the region presensitized with a PS, temoporfin in this example, because it has a significant absorption peak near 532 nm. Within 10 minutes a significant volume is ablated as the absorption of the target tissue has been considerably enhanced by the temoporfin (having an absorption peak in the green) and deeper photocoagulation and destruction occurs to enhance the area treated.

The enhanced destruction zone increases the safety of the procedure. The activation of the photosensitizer will result in an additional zone of 2 to 3 mm depth beyond that ablated and coagulated tissue that will be killed and eliminated in the days and weeks by the body, following the procedure. Thus ablated volume has been enhanced in a short treatment time with the presence of the photosensitizer, and treated tissue is further enhanced by the photocoagulation and destruction beyond the ablated zone, while maintaining greater safety for normal tissue because the latter processes are not due to thermal damage are relied upon towards the edges of the treated areas. Hence total treatment time is decreased and general anesthesia may be avoided. The procedure can now be carried out in an office setting.

To further enhance the treatment effect and deepen the zone to be eliminated by the body after the treatment ends, a 2 watt 652 nm laser can also be utilized simultaneously and concurrently with the KTP laser (532nm) radiation. The advantage here is the use of the 532 nm KTP laser to penetrate 1-2 mm into the prostate tissue causing thermal ablation and photoactivation of photosensitizer therein; further, the use of a 652 nm laser can activate the Temoporfin found deeper in the prostate tissue which can bring about photocoagulation and destruction of the deeper tissue with much less chance of thermal damage beyond the treated area.

### Example 2: Use of Laser and photosensitizer for tissue destruction

A liposomal temoporfin solution is administered by locally injecting 0.5 micrograms per gram of the prostate weight to be treated. (Thus for instance 20 micrograms is injected in a suitable solution if 40 g of prostate should be treated.) The injection can be done by utilizing a suitable needle with multiple holes in its circumference, (as shown for example in Figure 1) spaced for instance less than 3 mm apart along its axis, so as to ensure a good distribution of the active ingredient in the prostate tissue to be treated.

The presensitized prostate tissue is then irradiated with a wavelength around 652 nm. A side firing optical fiber is used for irradiating the prostate tissue. The optical fiber is introduced and positioned at the irradiation site using an endoscope. The activated photosensitizer induces photo-destruction in the prostate tissue. This Photodestruction is followed by lasing with higher wavelength laser having higher absorption in biological tissue like the 980 nm laser which is useful for absorption in blood-hemoglobin and water. A 980nm laser is used to bring about thermal destruction and photo-coagulation of hyperproliferative tissue minimizing blood loss and reducing edema in the treated region.

### Example 3: Highly absorbed thermal laser plus laser for PS absorption

A liposomal temoporfm solution is administered by locally injecting 0.5 micrograms per gram of the prostate weight to be treated. (Thus for instance 30 micrograms is injected in a suitable solution if 60 g of prostate should be treated.) The injection can be done by utilizing a suitable needle with multiple holes in its circumference, (as shown for example in Figure 1) spaced for instance less than 3 mm apart along its axis, or some other means, so as to ensure a good distribution of the active ingredient in the prostate tissue to be treated.

The presensitized prostate tissue is then irradiated with a wavelength around 652 nm. A side firing optical fiber is used for irradiating the prostate tissue. The optical fiber is introduced and positioned at the irradiation site using an endoscope. The activated photosensitizer induces photo-destruction in the prostate tissue. This photodestruction is followed by lasing with 1460 nm laser which has limited penetration into tissue. A 1460 nm laser is used to bring about thermal destruction/ablation of hyperproliferative tissue in limited depth of prostate, by minimizing blood loss and reducing edema in the treated region while deeper tissue coagulation is done by photoactivated Photosensitizer.

### Example 4: Using lasers with specific ablution, photo-coagulation and overlap with PS spectrums

A liposomal temoporfin solution is administered by locally injecting 0.5 micrograms per gram of the prostate weight to be treated. (Thus for instance 27.5 micrograms is injected in a suitable solution if 55 g of prostate should be treated.) The injection can be done by some appropriate means such as utilizing a suitable needle with multiple holes in its circumference, (as shown for example in Figure 1) spaced for instance less than 3 mm apart along its axis, so as to ensure a good distribution of the active ingredient in the prostate tissue to be treated.

The presensitized prostate tissue is then irradiated with a wavelength around 652 nm. A side firing optical fiber is used for irradiating the prostate tissue. The optical fiber is introduced and positioned at the irradiation site using an endoscope. The activated photosensitizer induces photo-destruction in the prostate tissue. This photodestruction is followed by lasing with more than one higher wavelength laser to bring about photoablation and coagulation in deeper regions of prostate. A 1460 nm laser is used for bring about highly precise tissue ablation with limited penetration into the tissue. A wavelength of 980 nm is used to photocoagulate somewhat deeper hyperplasic tissue of prostate with minimal blood loss and edema in treated region.

Using PS in combination with laser therapy can provide a double benefit, by increasing the absorption of the laser wavelength within the ablation/destruction zone to speed the ablation, and by photo-coagulating and destroying deeper tissue, regulated by wavelength choice and by the time delay from introduction of the PS and the actual irradiation. The loss of blood during the ablation will be considerably less with the attendant benefits in fiber control and patient recovery.

## Claims

1. A temoporfin for use in the treatment of diseased prostate tissue,
the treatment comprising delivery of the temoporfin and irradiation of the diseased prostate tissue containing the temoporfin using laser radiation provided by an optical fiber at a wavelength that is absorbed by said temoporfin,
wherein the laser radiation ablates and coagulates the prostate tissue nearest the optical fiber and photoactivates the temoporfin to destruct tissue deeper into the prostate, and
wherein a first laser wavelength of 532 nm, 980 nm, or 1460 nm is used to ablate prostate tissue and a second laser wavelength of 652 nm is used to activate the temoporfin.

2. The temoporfin for the use in the treatment of diseased prostate tissue according to claim 1,
wherein said temoporfin is delivered to said prostate using a special applicator needle inserted into a urethra channel within said prostate.

3. The temoporfin for the use in the treatment of diseased prostate tissue according to claim 2,
wherein said applicator needle has a plurality of openings along its transverse length to allow for a more rapid delivery of said medicament to tissues of said prostate.

4. The temoporfin for the use in the treatment of diseased prostate tissue according to claim 3,
wherein said applicator needle has multiple holes in its circumference which are spaced less than 3 mm apart along its axis.

5. The temoporfin for the use in the treatment of diseased prostate tissue according to claim 1,
wherein said optical fiber is inserted through an endoscope and wherein laser radiation ablates prostate tissue and photocoagulates deeper tissue for yielding minimal temperature damage to adjacent normal tissue.

6. The temoporfin for the use in the treatment of diseased prostate tissue according to claim 1,
wherein said optical fiber emits radiation through a side fire aperture.

7. The temoporfin for the use in the treatment of diseased prostate tissue according to claim 1,
wherein said temoporfin is delivered using a carrier selected from the group consisting of a liposome, pegylation, nanoparticles, and microspheres.

8. A PDT laser delivery system for temoporfin treatment, said laser delivery system comprising:
- an applicator needle insertable into an area of treatment for applying said temoporfin,
- an endoscope to guide and deliver said applicator needle to said area of treatment;
- an optical fiber to output laser radiation into said area of treatment,
wherein said laser radiation has a first laser wavelength of 532 nm, 980 nm or 1460 nm for ablation and/or coagulation;
**characterised in that**
- said laser radiation has a second laser wavelength of 652 nm for temoporfin activation and
- said applicator needle has a plurality of openings along its circumference to allow for a quicker application of said temoporfin to said area of treatment.

9. The laser delivery system of claim 8,
wherein said applicator needle has multiple holes in its circumference which are spaced on centers less than 3 mm apart along its axis.

10. The laser delivery system of claim 8,
wherein said optical fiber further comprises a side fire distal end.

## Patentansprüche

1. Temoporfin für die Verwendung bei der Behandlung von erkranktem Prostatagewebe,
wobei die Behandlung die Zufuhr von Temoporfin und die Bestrahlung des Temoporfin enthaltenden erkrankten Prostatagewebes mit Hilfe von Laserstrahlung umfasst, die durch eine optische Faser bei einer Wellenlänge bereitgestellt wird, die durch das Temoporfin absorbiert wird,
wobei die Laserstrahlung das Prostatagewebe in nächster Nähe zur optischen Faser ablatiert und koaguliert und das Temoporfin photoaktiviert, um Gewebe tiefer in der Prostata zu zerstören, und
wobei eine erste Laserwellenlänge von 532 nm, 980 nm oder 1460 nm dazu verwendet wird, das Prostatagewebe zu ablatieren und eine zweite Laserwellenlänge von 652 nm dazu verwendet wird, das Temoporfin zu aktivieren.

2. Temoporfin für die Verwendung bei der Behandlung von erkranktem Prostatagewebe gemäß Anspruch 1,
bei dem das Temoporfin mit Hilfe einer speziellen Anwendungsnadel zugeführt wird, die in einen Harnleiterkanal innerhalb der Prostata eingeführt wird.

3. Temoporfin für die Verwendung bei der Behandlung von erkranktem Prostatagewebe gemäß Anspruch 2,
bei dem die Anwendungsnadel eine Vielzahl von Öffnungen in Querrichtung aufweist, um eine schnellere Zufuhr des Medikaments zum Prostatagewebe zu ermöglichen.

4. Temoporfin für die Verwendung bei der Behandlung von erkranktem Prostatagewebe gemäß Anspruch 3,
bei dem die Anwendungsnadel mehrfache Löcher entlang ihres Umfangs hat, die weniger als 3 mm voneinander entfernt entlang ihrer Achse liegen.

5. Temoporfin für die Verwendung bei der Behandlung von erkranktem Prostatagewebe nach Anspruch 1,
bei dem die optische Faser durch ein Endoskop eingeführt wird und bei dem Laserstrahlung Prostatagewebe ablatiert und tieferes Gewebe photokoaguliert um einen minimalen Temperaturschaden am benachbarten normalen Gewebe zu erreichen.

6. Temoporfin für die Verwendung bei der Behandlung von erkranktem Prostatagewebe nach Anspruch 1,
bei dem die optische Faser Strahlung durch eine Öffnung für das seitliche Abstrahlen emittiert.

7. Temoporfin für die Verwendung bei der Behandlung von erkranktem Prostatagewebe nach Anspruch 1,
bei dem Temoporfin unter Verwendung eines Trägers zugeführt wird, der aus der Gruppe ausgewählt wird, die aus Liposom, Pegylierung, Nanoteilchen und Mikrosphären zusammengesetzt ist.

8. PDT-Laserzuführsystem für eine Temoporfinbehandlung, wobei das Laserzuführsystem umfasst:
- eine Anwendungsnadel, die in einen Behandlungsbereich zur Anwendung des Temoporfins einführbar ist,
- ein Endoskop, um die Anwendungsnadel zum Anwendungsbereich zu führen und diesem zuzuführen,
- eine optische Faser, um Laserstrahlung an den Behandlungsbereich auszugeben,
wobei die Laserstrahlung eine erste Laserwellenlänge von 532 nm, 980 nm oder 1460 nm für die Ablation und/oder Koagulation hat,
**dadurch gekennzeichnet, dass**
- die Laserstrahlung eine zweite Laserwellenlänge von 652 nm für die Aktivierung von Temoporfin umfasst und
- die Anwendungsnadel eine Vielzahl von Öffnungen entlang ihres Umfangs aufweist, um eine schnellere Anwendung des Temoporfins auf den Behandlungsbereich zu gestatten.

9. Laserzuführsystem nach Anspruch 8,
bei dem die Anwendungsnadel mehrfache Löcher entlang ihrem Umfang aufweist, die auf Zentren verteilt sind, die weniger als 3 mm entlang ihrer Achse voneinander entfernt sind.

10. Laserzuführsystem nach Anspruch 8,
bei dem die optische Faser ein seitlich abstrahlendes distales Ende aufweist.

## Revendications

1. Une témoporfine pour une utilisation dans le traitement d'un tissu prostatique malade,
le traitement comprenant l'administration de la témoporfine et l'irradiation du tissu prostatique malade contenant la témoporfine au moyen d'un rayonnement laser fourni par une fibre optique à une longueur d'onde qui est absorbée par ladite témoporfine,
dans laquelle le rayonnement laser ablate et coagule le tissu prostatique le plus proche de la fibre optique et photoactive la témoporfine de façon à détruire le tissu plus profondément dans la prostate, et
dans laquelle une première longueur d'onde laser de 532 nm, 980 nm ou 1460 nm est utilisée de façon à ablater le tissu prostatique et une deuxième longueur d'onde laser de 652 nm est utilisée de façon à activer la témoporfine.

2. La témoporfine pour l'utilisation dans le traitement d'un tissu prostatique malade selon la Revendication 1,
dans laquelle ladite témoporfine est administrée à ladite prostate au moyen d'une aiguille d'applicateur spéciale insérée dans un canal urétral à l'intérieur de ladite prostate.

3. La témoporfine pour l'utilisation dans le traitement d'un tissu prostatique malade selon la Revendication 2,
dans laquelle ladite aiguille d'applicateur possède une pluralité d'ouvertures le long de sa longueur transversale destinées à permettre une administration plus rapide dudit médicament à des tissus de ladite prostate.

4. La témoporfine pour l'utilisation dans le traitement d'un tissu prostatique malade selon la Revendication 3,
dans laquelle ladite aiguille d'applicateur possède une pluralité de trous sur sa circonférence qui sont espacés de moins de 3 mm le long de son axe.

5. La témoporfine pour l'utilisation dans le traitement d'un tissu prostatique malade selon la Revendication 1,
dans laquelle ladite fibre optique est insérée au travers d'un endoscope et dans laquelle un rayonnement laser ablate un tissu prostatique et photocoagule plus profondément un tissu de façon à produire un dommage de température minimal à des tissus normaux adjacents.

6. La témoporfine pour l'utilisation dans le traitement d'un tissu prostatique malade selon la Revendication 1,
dans laquelle ladite fibre optique émet un rayonnement au travers d'un orifice de tir latéral.

7. La témoporfine pour l'utilisation dans le traitement d'un tissu prostatique malade selon la Revendication 1,
dans laquelle ladite témoporfine est administrée au moyen d'un vecteur sélectionné dans le groupe se composant de liposome, pégylation, nanoparticules et microsphères.

8. Un système d'administration par laser PDT pour un traitement par témoporfine, ledit système d'administration par laser comprenant :
- une aiguille d'applicateur insérable dans une zone de traitement pour l'application de ladite témoporfine,
- un endoscope destiné à guider er à appliquer ladite aiguille d'applicateur à ladite zone de traitement,
- une fibre optique destinée à produire en sortie un rayonnement laser dans ladite zone de traitement,
ledit rayonnement laser possédant une première longueur d'onde laser de 532 nm, 980 nm ou 1460 nm pour une ablation et/ou une coagulation, **caractérisé en ce que**
- ledit rayonnement laser possède une deuxième longueur d'onde laser de 652 nm pour l'activation de la témoporfine, et
- ladite aiguille d'applicateur possède une pluralité d'ouvertures le long de sa circonférence destinées à permettre une application plus rapide de ladite témoporfine à ladite zone de traitement.

9. Le système d'administration par laser selon la Revendication 8, dans lequel ladite aiguille d'applicateur possède une pluralité de trous dans sa circonférence qui sont espacés de moins à 3 mm sur des centres le long de son axe.

10. Le système d'administration par laser selon la Revendication 8, dans lequel ladite fibre optique comprend en outre une extrémité distale de tir latéral.
